# EUROPEAN PATENT APPLICATION

(11) **EP 4 574 978 A1**
(43) Date of publication of application: **25.06.2025**
(21) Application number: 23461697.7
(22) Date of filing: 21.12.2023
(51) Int. Cl.: C12N 15/115

(54) **DNA APTAMER HAVING AFFINITY FOR PD-1 PROTEIN AND ITS USE**

(71) Applicant: UNIWERSYTET JAGIELLONSKI, 31-007 Kraków (PL)
(72) Inventor: Malicki, Stanislaw, Kraków (PL); Dubin, Grzegorz, Kraków (PL); Mydel, Piotr, Kraków (PL); Pucelik, Barbara, Kraków (PL); Zyla, Edyta, Kraków (PL); Benedyk-Machaczka, Malgorzata, Kraków (PL); Galan, Wojciech, Kraków (PL); Kaminska, Marta, Kraków (PL); Chruscicka-Smaga, Barbara, Kraków (PL); Sochaj-Gregorczyk, Alicja, Kraków (PL); Magiera-Mularz, Katarzyna, Kraków (PL); Czarna, Anna, Kraków (PL)
(74) Representative: Witek, Rafal

(57) **Abstract**

An ssDNA aptamer-based molecular probe capable of specific recognition of human PD1 receptor has been disclosed. The aptamer was selected using SELEX and tested for specificity and affinity. The potential of the probe in molecular imaging was determined in cell and organoid cultures.

## Description

The invention relates to an aptamer - a single-stranded nucleic acid molecule having affinity for the PD-1 (Programmed death receptor 1) protein and its use to produce a functional covalent complex containing either a fluorescent tag or an affinity substance. The invention also includes a functional covalent complex containing either a tag or an affinity substance and an oligonucleotide and its use in diagnostic imaging, particularly in cancer imaging.

PD-1 modulates the function of immune cells by providing inhibitory signals and constitutes the marker of immune exhaustion. Monitoring the level of PD-1 promises a useful diagnostic approach in autoimmune diseases and cancer.

Modern imaging techniques allow tracking of multifactorial changes in complex biological systems but require specialized molecular probes in the first place. Over the past ten years, oncology has seen a revolution thanks to immune checkpoint blocking antibodies that target the PD1/PD-L1 pathway. Accordingly, it was proposed that PD-L1 expression on tumor cells may be useful for prognostication and/or prediction. ^{1,2,3}, yet the strategy has a number of limitations ^{4,5} mainly because patients with tumors with minimal PD-L1 expression can still gain from anti-PD-L1 treatment. A more promising molecular marker is PD1. Expression of the receptor on immune cells enables monitoring of alterations due to fatigue. As a result, it guarantees valuable diagnostic data in a variety of clinical processes, such as autoimmune illnesses. ^{6, 7}, acute and chronic infection ⁸, sepsis ⁹ or cancer. According to a recent study by Xinyu Ren et al. ¹⁰ survival in triple-negative breast cancer could predicted by PD1 expression in tumor-infiltrating lymphocytes (TILs) rather than PD-L1 in tumor cells. Monitoring PD1 expression is a useful method for visualizing biological processes and a promising candidate for a diagnostic marker in a number of disorders.

PD1 tumor imaging differs from other types of imaging because it focuses on the environment of the tumor, specifically the immune infiltrating cells. ¹¹ which are visualized. Natarajan et. al. created an immune-based tracer to visualize tumor-inducible TILs (TILs) that express PD1. They labelled a murine anti-PD1 antibody with 64Cu. They successfully identified PD1- expressing murine TIL in transgenic mouse models of melanoma using PET (Positron Emission Tomography). This was the first demonstration that PET can be used for non-invasive imaging and in vivo measurement of PD1 expression. In another study, Hettich et al. ² developed high-resolution PET imaging radiotracers that allowed imaging of the PD1 receptor and PD-L1 ligand in cancers in immunocompetent mice. Another study reported the evaluation of the ⁸⁹Zr-labeled pembrolizumab antibody in several mouse models, including mice transplanted with human PBMC (peripheral blood mononuclear cells). ³. These initial concept has demonstrated the usefulness of this approach, but successful implementation requires additional work.

Probes designed for in vivo molecular imaging must provide high sensitivity, low background of noise, low toxicity, and relative stability ¹². Oligonucleotide aptamers emerge as highly promising candidates in this context ¹³, exhibiting both low immunogenicity high structural stability and low immunogenicity ¹⁴. Initial comparative analysis of antibodies and aptamers (both in *in vivo* and *in vitro* imaging) indicates that aptamers consistently provide superior advantages when compared to antibodies ^{15,16} though only broader development and use will reveal the true potential. Aptamers are currently being considered for optical imaging (fluorescence and bioluminescence), positron emission tomography (PET), magnetic resonance imaging (MRI), single photon emission tomography (SPECT), computed tomography (CT) and ultrasound (USA)¹³. Moreover, several clinical trials are investigating the diagnostic potential ¹³.

The technical problem to be solved by the invention is to provide a functional nucleotide sequence that binds specifically to the PD-1 protein, which could be used to obtain selective markers for use in cancer imaging across a broad spectrum of diagnostic techniques.

A first subject of the invention is an aptamer with affinity for the PD-1 protein comprising a nucleotide sequence of at least 95% identity to the sequence of SEQ. 1, preferably comprising the sequence shown as SEQ.1, particularly preferably having this sequence.

Determination of the sequence identity is possible using standard DNA sequencing methods and bioinformatic sequence analysis methods, whereby the introduction of single changes in the nucleotide sequence of the aptamer does not lead to a change in its conformation that determines the match to the PD-1 target protein. Since the aptamer is made of 90 nucleotides, it is not possible to study all combinations of single changes for all nucleotides.

A second subject of the invention is the use of the aptamer of the invention to produce a functional covalent complex comprising a fluorescent tag or an affinity substance and the aptamer of the invention.

In a preferred embodiment of the invention, the fluorescent tag is selected from the group comprising: fluorescein, , or Cy5.5. According to the invention, the tag is to be understood as any known substance suitable and used for oligonucleotide labelling by its covalent attachment to the oligonucleotide. An exemplary tag suitable for obtaining the covalent complex of the invention is fluorescein.

In a further preferred embodiment of the invention, the affinity substance is biotin. According to the invention, the affinity substance is to be understood as any known substance suitable for isolation by affinity chromatography. An exemplary substance suitable for obtaining the covalent complex of the invention is biotin.

A further subject of the invention is a functional covalent complex comprising the tag or the affinity substance and the aptamer of the invention.

In a preferred embodiment of the invention, the fluorescent tag is selected from the group comprising: fluorescein, or Cy5.5.

In another preferred embodiment of the invention, the affinity substance is biotin.

Another subject of the invention is the use of the functional covalent complex of the invention in diagnostics, in particular in diagnostic imaging, preferably in tumour imaging.

The present invention is based on the development of an ssDNA aptamer-based molecular probe capable of specific recognition of human PD1 receptor. The aptamer was selected using SELEX, its sequence was further optimized, and the affinity and specificity were determined in biochemical assays. The aptamer was converted into a fluorescent probe and its potential in molecular imaging was demonstrated in a culture of human cells overexpressing PD1 and murine pancreatic organoids / immune cells mixed co-culture model. The provided aptamers are suitable probes for imaging of PD-1 expressing immune cells even in complex cellular models and may find future utility as diagnostic tools.

Among the available diagnostic imaging techniques in which the aptamer, which is the subject of the present invention, can be used are: optical imaging (fluorescence and bioluminescence), magnetic resonance imaging, positron emission tomography, single photon emission tomography, computed tomography and ultrasonography. As well as other techniques that can be used to determine PD-1 protein levels on the cell surface, whereby imaging can relate to cancer and other diseases in which changes in PD-1 protein levels are involved.

The object of the invention is made apparent in the examples and the drawing, which is not limiting to the scope of the application, wherein:

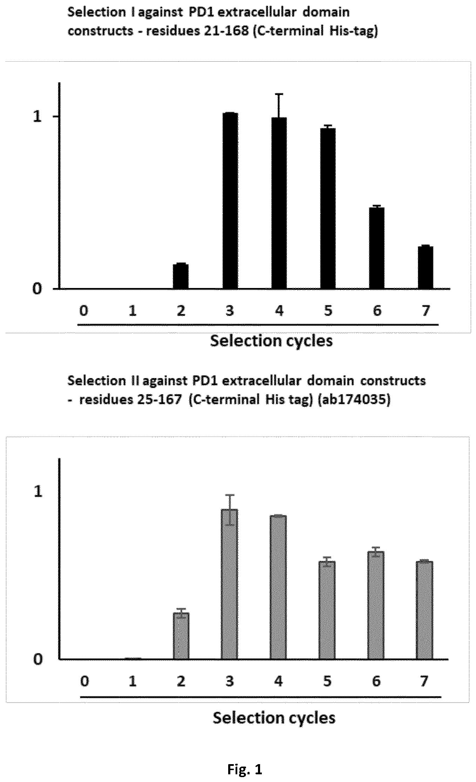

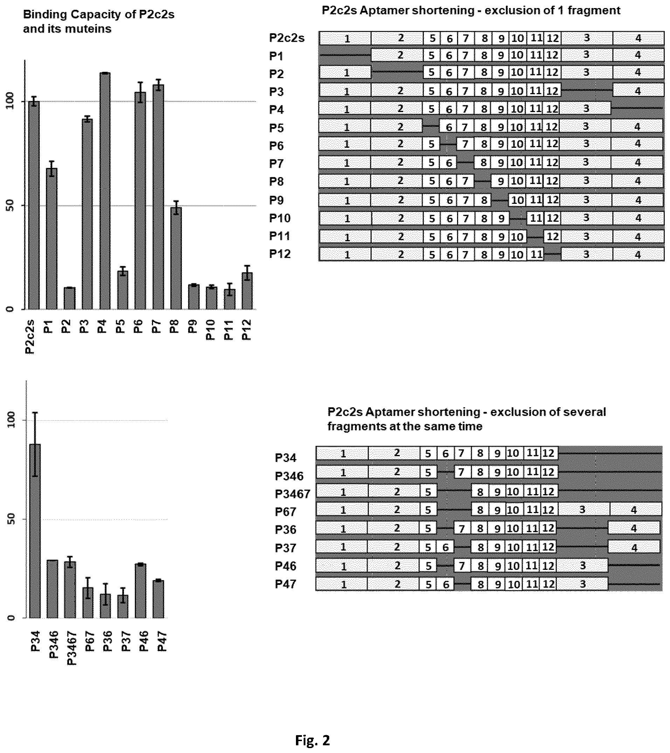

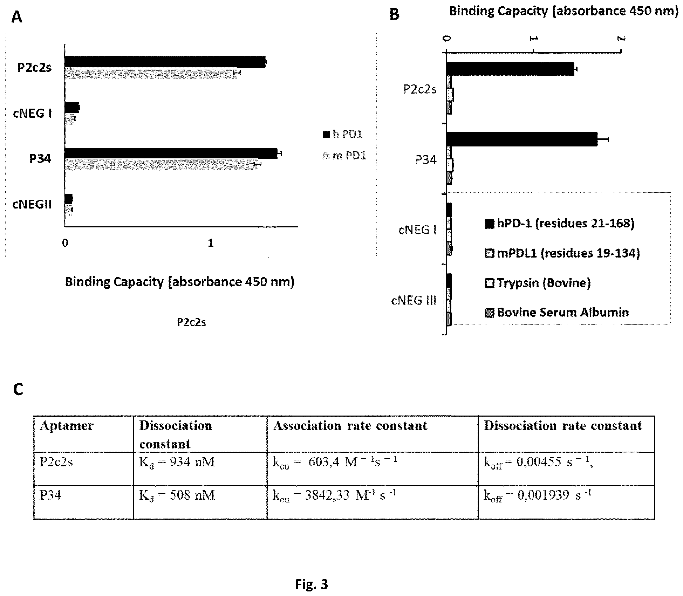

Figure 1 Selection of PD1 targeting aptamers. (A, B) Enrichment of ssDNA pool with aptamers recognizing the extracellular domain of PD1 (constructs indicated) determined using ELISA. Figure 2 shows the identification of the minimal P2c2 fragment responsible for the interaction with PD1, later named as p34. Positional Scan: Tested combinations of regions identified as nonessential for binding in positional scan (the binding ability of the muteins devoid of tested regions to the extracellular domain of the PD1 protein was demonstrated using the ELISA test).Figure 3 Figure 3 presents affinity and specificity of P2c2s and its short version - P34. A - Selective binding of indicated aptamers to human PD1 (hPD1, residues 21-168, C-terminal His-tag) and to mouse PD1 (mPD1, residues 25-167, C-terminal His-tag) as determined using ELISA. cNEG1 and cNEG2-non-specific sequences. C-The table shows the SPR measurement data characterizing binding of tested aptamers to immobilized PD1.

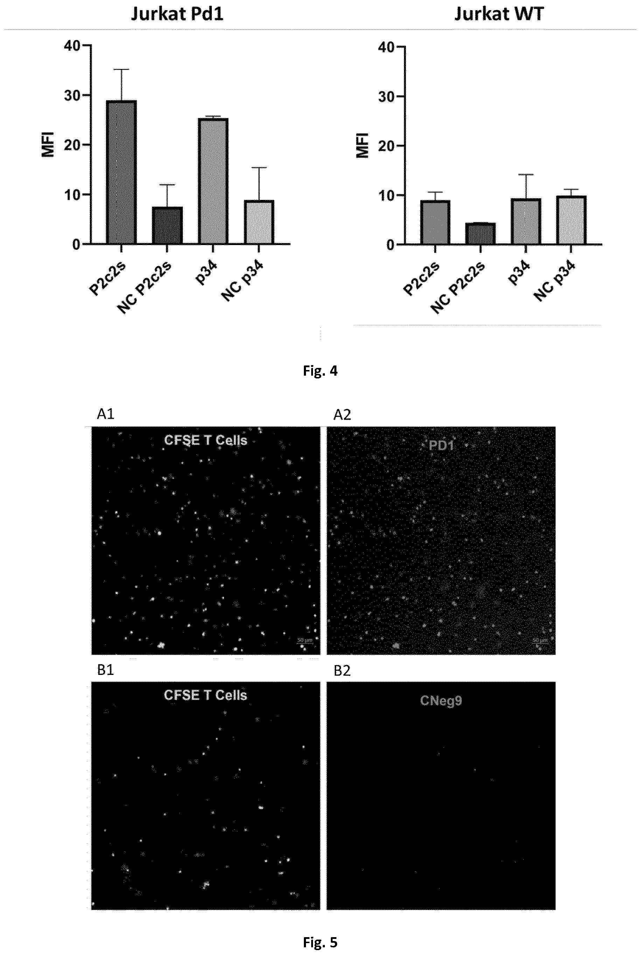

Figure 4 presents results which confirm that p2c2s and p34 recognize PD1 at the cell surface. Cells were incubated with tested FITC-labeled aptamers and the labeling was evaluated using flow cytometry. The graph presents quantitative representation of flow cytometry data for Jurkat T cells stably expressing human PD1 and Wild-type Jurkat cells (NC1, NC2 were non-specific aptamers corresponding in length to P2c2s and p34, respectively).
Figure 5 presents results which confirm that P34 (short version of P2c2s) specifically recognizes PD1 at the surface of immune cells in KPC organoid / T-cell co-culture. Ex vivo live staining (A1, A2) T-cells labeled with CSFE and (B1) PD1 expressed on T-cells (P34-TxRd staining) (B2) PD1 expressed on T-cells (NC-TxRd staining).

### Example 1. Selection of aptamers.

### PD1 purification

Construct of the extracellular domain of human PD1 (residues 21-168 with C-terminal His-tag) was expressed and purified as described previously ²⁷. The protein was expressed in *E. coli* BL21 (DE3) as inclusion bodies. Cells were cultured in LB medium at 37 °C and induced with 1 mM IPTG (Isopropyl-β-D-thiogalactoside) at OD600 = 1.0. After further 3-hours incubation at 37 °C, the cells were collected and sonicated in phosphate-buffered saline (PBS). Inclusion bodies were recovered by centrifugation, washed twice with 50 mM Tris-HCl pH 8.0, 200 mM NaCl, 0.5% Triton X-100, 10 mM EDTA and 10 mM 2-mercaptoethanol followed by washing with the same buffer excluding the detergent. The inclusion bodies were dissolved in 50 mM Tris pH 8.0, 6M GuHCl, 200 mM NaCl, and 10 mM 2-mercaptoethanol and refolded by drop-wise dilution into 0.1 M Tris pH 8.0, 0.4 M L-Arg hydrochloride, 2 mM EDTA, 5 mM cystamine and 0.5 mM cysteamine. After refolding, protein was dialyzed 3 times against 10 mM Tris pH 8.0 containing 20 mM NaCl, concentrated, and further purified by gel filtration on Superdex 75 (GE Healthcare, Chicago, Illinois, USA) in buffer containing; 25 mM Na₂HPO₄, 25 mM NaH₂PO₄ pH 6.4, 100 mM NaCl, 5 mM MgCl₂ and 10 mM KCI or 10 mM Tris pH 8.0 containing 20 mM NaCl. The proteins were stored for further experiments as 0.2 mg/ml stocks at -80°C. Additionally, recombinant human PD1 containing a C-terminal His tag (amino acids 25-167) (ab174035) and recombinant mouse PD1 (amino acids 21-167) with a His tag at the C-terminus (ab180051) were acquired from Abcam, Cambridge, UK.

### In vitro selection of aptamers

The single-stranded DNA (ssDNA) library (N50), denoted as N50, consisted of a random section of 50 nucleotides flanked by fixed 20-nucleotide primer sequences (5'-CATGCTTCCCCAGGGAGATG-N50-GAGGAACATGCGTCGCAAAC-3') synthesized at 0.2 µM scale and purified by HPLC (IBA, Göttingen, Germany). Before each subsequent selection cycle ssDNA was renatured by incubation for 5 min at 92 ° C, 10 min 4 ° C and 15 min RT. Prior to productive selection, aptamers interacting with the carrier resin were removed by incubation with uncoated beads. Aptamers were selected independently against PD1 extracellular domain constructs, one and the other covering residues 25-167 (obtained from Abcam, UK, ab174035), both tagged with C-terminal histidine tags. The target protein was immobilized using Dynabeads ^{™} His-Tag Isolation and Pulldown kit from Thermo Fisher Scientific, following the manufacturer's instructions. The beads were washed with selection buffer (PBS pH 7.4 with 5mM MgCl₂, 10mM KCI, and 0.01% Tween 20) and suspended in binding buffer (selection buffer with 40-120µg/ml yeast tRNA and 125 µg/ml BSA). The ssDNA library was introduced and incubated at 24°C for 20 minutes with stirring. To heighten selective pressure, in successive cycles, bead quantity decreased gradually, while the competitor concentration (yeast tRNA) increased. (Figure 1). Following the incubation, the beads underwent a rinsing process and then were suspended in distilled water (dH2O). Subsequently ssDNA was amplified through PCR. A PCR mixture containing 1 µM of unmodified ss50_For primer (5'-CATGCTTCCCCAGGGAGATG-3') and 5'-phosphorylated ss50_Rev primer (5'-GTTTGCGACGCATGTTCCTC-3'), 0.5 mM deoxynucleotide triphosphates (dNTPs), 2.5 mM MgCl₂, and 1.25U/100 µl of Taq polymerase (Thermo Fisher Scientific, Waltham, USA) was prepared (in a volume of 400 µl) and combined with a beads containing immobilized protein and attached aptamers. The PCR process involved 30 cycles, including denaturation at 95 °C for 2 min, annealing at 53 °C for 30 s, and extension at 72 °C for 30 s, followed by a final polishing step at 72 °C for 5 min.The PCR products were isolated using a phenol-chloroform-isoamyl alcohol solution from Sigma-Aldrich, St.Louis, USA, and then precipitated with ethanol at -20°C. The resulting DNA pellet was dissolved in distilled water (dH2O). This double-stranded DNA (dsDNA) was transformed into the relevant single-stranded DNA (ssDNA) by treating it with λ exonuclease from Thermo Fisher Scientific, Waltham, USA (in 500 µl solution containing 100 U of enzyme with gentle shaking). The ssDNA was then extracted using phenol-chloroform-isoamyl alcohol. To track the enrichment of PD1 binding aptamers in the following selection rounds, ELISA was employed (details below). After the 5th, 6th, and 7th selection cycles, the aptamer pools were subjected to analysis using Next-Generation Sequencing (NGS) by Genomed S. A., Warszawa, Poland.

### Sequence analysis

FASTQ files with demultiplexed sequences were trimmed for adapters and reversed when necessary. Sequences shorter than 10 nucleotides were removed. Identical sequences were grouped and represented by a common sequence (compressed_seq). The top 10 compressed_seqs were clustered using k-means. Optimal clusters were determined via the silhouette method (2<=k<=4) and aligned with ClustalW. Alignments were processed with dnadist and neighbour from PHYLIP to create (pseudo-)phylogenetic trees showing relationships between compressed_seqs.

### Example 2. Preparation of covalent complexes of the invention

Covalent complexes containing aptamer and a functional substance selected from the group: fluorescent tag were ordered from a company specializing in the synthesis of modified and unmodified oligonucleotides (Millipore), where fluorescein (fluorescein isothiocyanate), Cyanine 5.5 (Cy5.5), as a tag, or biotin, as an affinity substance, were introduced into the aptamer molecules by standard chemical synthesis methods (C. K. Brush "Fluorescein Labelled Phosphoramidites", U.S. Patent 5,583,236, 1996); R. T. Pon "A long chain biotin phosphoramidite reagent for the automated synthesis of 5'-biotinylated oligonucleotides", Tetrahedron Lett, 1991).

Unmodified aptamers or pools (mixtures of aptamers) were only used during selection. All analyses conducted to characterise the developed molecules were carried out using aptamers modified with (a tag): biotin or fluorescent tag.

### Example 3 Determination of specificity and functionality of the obtained aptamers and covalent complexes

In the ELISA binding assay, human PD1 at a concentration of 10 µg/ml in PBS was coated onto a microtiter plate (Nunc, Rochester, NY, USA) for 1 hour. After removing unbound PD1 through washing, biotinylated aptamers were introduced in the selection buffer and incubated. To assess nonspecific binding, control ssDNA sequences were used. Subsequent to washing, streptavidin-HRP was applied, and after another round of washing, the substrate was added. The reaction was halted, and the absorbance at 450 nm was measured. Similar experiments were conducted using unrelated proteins such as mouse extracellular domain of PD-L1, bovine trypsin, and bovine serum albumin.

### Binding kinetics

Surface plasmon resonance (SPR) was employed to characterize the binding kinetics of P2c2 and P34 to the PD1 (extracellular domain: residues 21-168; C-terminal His-tag). Biotinylated aptamers, initially prepared as a 5 nM stock in HBS-P buffer (0.01 M HEPES pH 7.4, 0.15 M NaCl, 0.005% v/v Surfactant P20, and 5 mM MgCl₂) were immobilized at a density of around 700 RU on an SA chip (GE Healthcare, Chicago, Illinois, USA) as per the manufacturer's instructions. The binding kinetics were assessed in a PD1 aptamer assay buffer (25 mM Na₂HPO₄, 25 mM NaH₂PO₄ pH 6.4, 100 mM NaCl, 5 mM MgCl₂, and 10 mM KCI) using Biacore X100 (GE Healthcare, Chicago, Illinois, USA). Chip regeneration in multi-cycle kinetics was accomplished with a brief pulse of 2 M KCI for 20 seconds. The obtained binding data were then analyzed using Biacore X100 Evaluation software, employing the Langmuir 1:1 binding model.

### Cell binding assay (flow cytometry)

Jurkat T-cells (wild type and overexpressing PD1) were cultured in RPMI 1640 medium with 10% FBS at 37°C with 5% CO₂. Cells expressing hPD1 were maintained with selection antibiotics (hygromycin B and G418). Aptamers (P2c2s, P34, and nonspecific sequences) labelled with 5'-FAM, 5'-Texas Red, or 5'-cyanine were used. Cells were blocked (in Hanks' Balanced Salt solution (HBSS) supplemented with 1 mg/ml dextran (Merck Millipore , Darmstadt, Germany) and 1 mg/ml salmon sperm DNA (Merck Millipore, Darmstadt, Germany) for 30 min at 37°C) and labelled with aptamers (0-250nM) in HBSS with MgCl₂ and KCI, incubated at 37°C in the dark with shaking. Flow Cytometry (BD FACSCalibur) was used to analyze cells, The binding of fluorophore-conjugated aptamers to the target protein was demonstrated as a percentage of fluorescent positive cells using CellQuestPro software. Human PD1 antibodies (clone J105, Thermo Fisher Scientific, Waltham, MA, USA) were positive controls, and nontransfected Jurkat cells served as negatives.

### Ex vivo aptamer analysis in KPC organoid / T-cell co-culture

In the study, we utilized KPC mice (Pdx1-Cre; Kras+/LSL-G12D; Trp53+/LSL-R172H) bearing pancreatic tumors. Mice aged between 6 to 8 weeks were procured from Charles River Laboratory. To isolate mouse pancreatic tumor cells from mice older than 8 weeks, a previously established method was employed. This involved mincing the pancreatic tumors and subjecting them to enzymatic dissociation using with 5 mg/ml Collagenase type XI (Gibco, Thermo Fisher Scientific, Waltham, MA, USA), 1 mg/ml Dispase (Gibco, Thermo Fisher Scientific, Waltham, MA, USA), 1% FBS (Gibco, Thermo Fisher Scientific, Waltham, MA, USA) in DMEM medium (Gibco, Thermo Fisher Scientific, Waltham, MA, USA) at 37 °C for a maximum of 16 hrs. The isolated material was incubated 10 min with TrypLE (Gibco, Thermo Fisher Scientific, Waltham, MA, USA) at 37 °C , embedded into growth factor-reduced Matrigel (Corning), and cultured in mouse complete medium (AdDMEM/F12 (Gibco) supplemented with 1% penicillin/streptomycin (Gibco), 1% GlutaMAX (Gibco), 10 mM HEPES (Gibco), 1:50 B27 supplement (Gibco), 1.25 mM N-Acetylcysteine (Sigma Aldrich, Darmstadt, Germany), 10% (vol/vol) Rspo1-conditioned media,, 10 nM recombinant human-gastrin I (Tocris), 10 mM Nicotinamide (Sigma Aldrich, Darmstadt, Germany), 50 ng/ml recombinant mouse EGF (Gibco), 100 ng/ml recombinant human FGF10 (Peprotech, London, UK), 0.5 µM A83-01 (Tocris), and 100 ng/ml recombinant human Noggin (Peprotech, London, UKThe medium was changed twice a week. All animal studies were authorized by the Animal Care and Use Committee at SCUT, and rigorous measures were taken to minimize any distress or discomfort experienced by the animals during the experiments.KPC organoids were cultured with CFSE-labelled T cells. Before staining with aptamers, the organoids were treated 30 min. with a blocking buffer (HBSS + 0.1 mg/mL dextran + 1 mg/mL salmon sperm DNA). Then, P34-TxR aptamer or a negative control aptamer (both at concentration 50 nM) labeled with TxR were introduced and incubated with the organoids for 6 hours. After this incubation , the organoids were washed using HBSS through pipetting. Subsequently, images were captured using a confocal fluorescence microscope (Carl Zeiss).

### Results

### Identification and characterization of ssDNA aptamers targeting human PD1

Aptamers were selected for two recombinant PD1 forms: i) spanning residues 21-168 and ii) residues 25-167, covering the substantial portion of the human PD1 extracellular domain. To prevent the selection of aptamers specific to a particular protein construct, both protein variants were employed. The selection process followed the SELEX procedure, involving a gradual increase in selective pressure. ¹⁷. After each selection cycle, binding to the target protein was assessed via ELISA. Maximum saturation occurred consistently after the 4th cycle (Figure 1). Subsequent cycles showed a gradual decrease in the specific signal. Aptamer pools from the 5th cycle onwards were analyzed using next-generation sequencing (NGS). The sequences obtained were grouped into similar clusters, and the frequency of specific sequences within clusters was examined. Selection against the PD1 extracellular domain (21-168) produced two aptamer clusters (P1c and P2c). Selection against the PD1 extracellular domain (25-167) resulted in a single cluster, corresponding to cluster P2c from the previous selection. This demonstrates that the selection converges at the PD1 core, indicating no significant differences in protein construct design. Results for cycles 5, 6, and 7 were qualitatively similar (identical sequences observed with different relative frequencies). The declining binding capacity of aptamer pools after cycle 5 was due to an increasing presence of low-specificity sequences and non-specific amplification products containing only flanking sequences.The ELISA analysis was employed to assess the affinity of the selected aptamers for PD1. Cluster P1c, specific to the selection against PD1 (residues 21-168), included sequences with minimal or no affinity to PD1. On the other hand, cluster P2c, common to selections against both PD1 constructs, exhibited noticeable binding. Within cluster P2c, individual sequences showed similar binding capacities, indicating minor differences in their nucleotide sequences. The strong affinity of P2c cluster aptamers, for PD1 compared to P1c indicated P2c2s specificity. The dominant sequence within the P2c cluster, namely the P2c2 aptamer, was chosen for further analysis.In this study, aptamers were generated from a library with a 50-nucleotide randomized region. Previous research has shown that not all nucleotides within aptamers are crucial for maintaining binding to the target protein. Consequently, to determine the minimal sequence we utilized positional scanning (mapping) of P2c2s. ¹⁸ To identify the binding regions on the extracellular domain of PD1, P2c2s was analyzed (Fig. 2), the P2c2s sequence was divided into 12 segments (each comprising 6-10 nucleotides). Variants were synthesized, each with a single segment deleted, and tested using ELISA for their interaction with PD1. Deletion of flanking segments 3 and 4, as well as segments 6 and 7 within the variable region, did not impact the binding, while other variants showed compromised binding (Fig. 2). Using these findings, P2c2s variants were designed, eliminating multiple segments identified as unnecessary for binding in the positional scanning. Simultaneous removal of segments 3 and 4 did not affect the aptamer's (P34) affinity for PD1, whereas all other tested combination variants resulted in loss of activity (Fig. 2B). Further analysis was focused on the parent aptamer (P2c2s) and the P34 variant.

### P2c2s and P34 recognize PD1 with high affinity and specificity

To confirm the selectivity of the aptamers chosen, the binding capacity of the P2c2s and P34 with nonspecific sequences containing flanking fragments and (AAAACCCCTTTTGGGG) repeat was compared. cNEG1 and cNEG2 (corresponding to P2c2s and P34, respectively) showed no significant binding, in contrast to the high signal observed for the parent aptamers (Figure 3). To assess P2c2s and P34's specificity, we tested their binding to human PD1 as well as other selected proteins.. None of the tested sequences, including P2c2s, P34, and the non-specific ones, showed any significant affinity for mouse PD-L1 (residues 19-134), bovine serum albumin, or trypsin. Crucially, both P2c2s and P34, relevant to our study, demonstrated specific recognition of the human extracellular domain of PD1 and the mouse extracellular domain of PD1 (which shares high homology with the human domain), highlighting their high level of specificity.After confirming P2c2s and P34's specificity, the binding kinetics of the aptamers was determined using SPR (Fig. 3). Both aptamers exhibited high nanomolar affinity for the extracellular domain of human PD1 (21-168) . Removing non-binding regions in P34 nearly doubled its affinity compared to P2c2s. This rise in affinity was evident through higher kon rates and lower k_{off} rates.

### The assess of the aptamers' capability to specifically identify PD1 on cell surfaces.

The staining of Jurkat-PD1 cells with overexpressing of the PD1 receptor and wild-type cells with negligible expression of PD1 was compared. Safe aptamer concentrations were determined through LDH and MTT analyses. FITC-labeled P2c2s and P34 effectively stained Jurkat cells overexpressing PD1 while showing no significant staining on wild-type cells (Fig. 4). Although there was some non-specific binding observed with PD1-unrelated sequences (NC1, NC2), the signal was markedly lower compared to the specific aptamers of interest.

### P34 successfully identified PD1 expression in the organoid tumor model

To assess P34's ability in a more physiologically relevant setting, the complex three-dimensional co-culture system involving tumor-derived murine pancreatic organoids (KPC) and T-cells was utilized. Organoids were exposed to Texas Red-labeled P34 and analyzed using confocal microscopy. The labeling displayed a speckled pattern that precisely aligned with CFSE-labeled T-cells (Fig. 5) and not other organoid components. These findings confirm P34's selective labeling of PD1 on immune cell surfaces within this intricate three-dimensional organotypic co-culture system.

### Bibliography

1. Natarajan, A. et al. Novel Radiotracer for ImmunoPET Imaging of PD-1 Checkpoint Expression on Tumor Infiltrating Lymphocytes. Bioconjugate chemistry 26, 2062-2069 (2015).
2. Hettich, M., Braun, F., Bartholomä, M. D., Schirmbeck, R. & Niedermann, G. High-Resolution PET Imaging with Therapeutic Antibody-based PD-1/PD-L1 Checkpoint Tracers. Theranostics 6, 1629-1640 (2016).
3. England, C. G. et al. Preclinical Pharmacokinetics and Biodistribution Studies of 89Zr-Labeled Pembrolizumab. Journal of nuclear medicine: official publication, Society of Nuclear Medicine 58, 162-168 (2017).
4. Maleki Vareki, S., Garrigós, C. & Duran, I. Biomarkers of response to PD-1/PD-L1 inhibition. Critical reviews in oncology/hematology 116, 116-124 (2017).
5. Davis, A. A. & Patel, V. G. The role of PD-L1 expression as a predictive biomarker: an analysis of all US Food and Drug Administration (FDA) approvals of immune checkpoint inhibitors. Journal for immunotherapy of cancer 7, (2019).
6. Álvarez-Sierra, D. et al. Analysis of the PD-1/PD-L1 axis in human autoimmune thyroid disease: Insights into pathogenesis and clues to immunotherapy associated thyroid autoimmunity. Journal of autoimmunity 103, (2019).
7. MR, J. et al. Downregulation of Immunosuppressive Molecules, PD-1 and PD-L1 but not PD-L2, in the Patients with Multiple Sclerosis. Iranian journal of allergy, asthma, and immunology 15, 296-302 (2016).
8. Jubel, J. M., Barbati, Z. R., Burger, C., Wirtz, D. C. & Schildberg, F. A. The Role of PD-1 in Acute and Chronic Infection. Frontiers in immunology 11, (2020).
9. Wilson, J. K., Zhao, Y., Singer, M., Spencer, J. & Shankar-Hari, M. Lymphocyte subset expression and serum concentrations of PD-1/PD-L1 in sepsis - pilot study. Critical care (London, England) 22, (2018).
10. Ren, X. et al. PD1 protein expression in tumor infiltrated lymphocytes rather than PDL1 in tumor cells predicts survival in triple-negative breast cancer. Cancer biology & therapy 19, 373-380 (2018).
11. Ehlerding, E. B., England, C. G., McNeel, D. G. & Cai, W. Molecular Imaging of Immunotherapy Targets in Cancer. Journal of nuclear medicine: official publication, Society of Nuclear Medicine 57, 1487-1492 (2016).
12. Chakravarty, R., Goel, S. & Cai, W. Nanobody: the 'magic bullet' for molecular imaging? Theranostics 4, 386-398 (2014).
13. Yoon, S. & Rossi, J. J. Targeted Molecular Imaging Using Aptamers in Cancer. Pharmaceuticals (Basel, Switzerland) 11, (2018).
14. Que-Gewirth, N. S. & Sullenger, B. A. Gene therapy progress and prospects: RNA aptamers. Gene therapy 14, 283-291 (2007).
15. De Castro, M. A. G., Hobartner, C. & Opazo, F. Aptamers provide superior stainings of cellular receptors studied under super-resolution microscopy. PloS one 12, (2017).
16. Melancon, M. P. et al. Selective uptake and imaging of aptamer- and antibody-conjugated hollow nanospheres targeted to epidermal growth factor receptors overexpressed in head and neck cancer. ACS nano 8, 4530-4538 (2014).
17. Malicki, S. et al. Imaging of Clear Cell Renal Carcinoma with Immune Checkpoint Targeting Aptamer-Based Probe. Pharmaceuticals (Basel, Switzerland) 15, (2022).
18. Gao, S., Zheng, X., Jiao, B. & Wang, L. Post-SELEX optimization of aptamers. Analytical and bioanalytical chemistry 408, 4567-4573 (2016).
19. Belorgey, D. & Bieth, J. G. DNA binds neutrophil elastase and mucus proteinase inhibitor and impairs their functional activity. FEBS letters 361, 265-268 (1995).
20. AN, N. et al. Whole body PD-1 and PD-L1 positron emission tomography in patients with non-small-cell lung cancer. Nature communications 9, (2018).
21. SL, T. et al. Safety, activity, and immune correlates of anti-PD-1 antibody in cancer. The New England journal of medicine 366, 2443-2454 (2012).
22. Ribas, A. & Wolchok, J. D. Cancer immunotherapy using checkpoint blockade. Science 359, 1350-1355 (2018).
23. X, G. et al. Development of 99m Tc-conjugated JS001 antibody for in vivo mapping of PD-1 distribution in murine. Bioorganic & medicinal chemistry letters 29, 2178-2181 (2019).
24. H, H. et al. Evaluation of 124 I-JS001 for hPD1 immuno-PET imaging using sarcoma cell homografts in humanized mice. Acta pharmaceutica Sinica. 8 10, 1321-1330 (2020).
25. Broos, K. et al. Noninvasive imaging of the PD-1:PD-L1 immune checkpoint: Embracing nuclear medicine for the benefit of personalized immunotherapy. Theranostics 8, 3559-3570 (2018).
26. Blackburn, S. D. et al. Coregulation of CD8+ T cell exhaustion by multiple inhibitory receptors during chronic viral infection. Nature immunology 10, 29-37 (2009).
27. Zak M.K, Grudnik P., Guzik K., Zieba JB, Musielak B., Dömling A., Dubin G., and H. A. Structural basis for small molecule targeting of the programmed death ligand 1 (PD-L1). Oncotarget. May 24; 7, 30323-30335 (2016).
28. Bartfeld, S. & Clevers, H. Organoids as Model for Infectious Diseases: Culture of Human and Murine Stomach Organoids and Microinjection of Helicobacter Pylori. Journal of visualized experiments : JoVE 2015, (2015).

SEQUENCE LISTING

Seq. Id. Nr. 1

P2c2s

CATGCTTCCCCAGGGAGATGCGGGGGGTGGGAGGGTGGAGCAGCTGTGGTTGGTGTGGTTGGACCCTGGAG
GAACATGCGTCGCAAAC

Seq. Id. Nr. 2

P 34

CATGCTTCCCCAGGGAGATGCGGGGGGTGGGAGGGTGGAGCAGCTGTGGTTGGTGTGGTTGGACCCTG

## Claims

1. An aptamer having affinity for the PD-1 protein comprising a nucleotide sequence having at least 95% identity to the sequence SEQ. 1.

2. The aptamer of claim 1, **characterised in that** it comprises the sequence shown as Seq.ld. Nr. 1 or Seq.ld. Nr. 2.

3. Use of the aptamer defined in claims 1-2 to produce a functional covalent complex, wherein the complex comprises a fluorescent tag or an affinity substance and the oligonucleotide defined in claims 1-2.

4. The use of claim 3, **characterised in that** the fluorescent tag is selected from the group comprising: fluorescein, preferably fluorescein isothiocyanate, or Cy5.5.

5. The use of claim 3, **characterised in that** the affinity substance is biotin.

6. A functional covalent complex comprising a tag or an affinity substance and the oligonucleotide defined in claims 1-2.

7. The functional covalent complex of claim 6, **characterised in that** the fluorescent tag is selected from the group comprising: fluorescein or Cy5.5.

8. The functional covalent complex of claim 6, **characterised in that** the affinity substance is biotin.

9. The aptamer defined in claims 1-2 or the functional covalent complex defined in claims 6-8 for use in diagnostics, particularly in: optical imaging such as fluorescence or bioluminescence, positron emission tomography (PET), magnetic resonance imaging (MRI), single-photon emission tomography (SPECT), computed tomography (CT), and ultrasound (US), preferably in cancer imaging.
